# EUROPEAN PATENT APPLICATION

(11) **EP 4 825 396 A1**
(43) Date of publication of application: **21.10.2026**
(21) Application number: 24902898.6
(22) Date of filing: 12.12.2024
(51) Int. Cl.: A61K 39/395, C07K 16/38, A61P 7/04

(54) **TFPI ANTIBODY USED IN THE TREATMENT OF HEMOPHILIA**

(30) Priority: 13.12.2023 CN 202311714133
(71) Applicant: Suzhou Alphamab Co., Ltd, Jiangsu 215125 (CN)
(72) Inventor: XU, Ting, Suzhou, Jiangsu 215125 (CN); DONG, Yanrong, Suzhou, Jiangsu 215125 (CN); WANG, Pilin, Suzhou, Jiangsu 215125 (CN); YIN, Qun, Suzhou, Jiangsu 215125 (CN); SUN, Wei, Suzhou, Jiangsu 215125 (CN)
(74) Representative: Henderson, Helen Lee
(86) International application number: PCT/CN2024/138816
(87) International publication number: WO 2025/124481

(57) **Abstract**

Provided is a use of a TFPI antibody in the treatment of hemophilia. Specifically, using the TFPI antibody can significantly reduce the bleeding frequency of hemophilia A and hemophilia B patients with or without inhibitors.

## Description

The present application claims priority to Chinese Patent Application 2023117141330 filed on December 13, 2023, which is incorporated herein by reference in its entirety.

### TECHNICAL FIELD

The present application relates to the field of biopharmaceuticals and particularly to a TFPI antibody for treating hemophilia.

### BACKGROUND

Hemophilia is an X-chromosome-linked recessive hereditary bleeding disease and is classified into hemophilia A and hemophilia B. The former is manifested in a deficiency of factor VIII (FVIII) and accounts for 80%-85%, and the latter is manifested in a deficiency of factor IX (FIX) and accounts for 15%-20%. Among males, the incidence of hemophilia A is about 1/5000, and the incidence of hemophilia B is about 1/25,000. Female hemophilia patients are extremely rare. Clinically, hemophilia mainly presents as joint, muscle, and deep tissue bleeding, and can also involve gastrointestinal, urinary tract, and central nervous system bleeding, persistent bleeding after tooth extraction, delayed bleeding following trauma or surgery, etc. Recurrent bleeding and delayed treatment can lead to joint deformities and/or pseudotumor formation, severely impacting the patient's quality of life. Severe bleeding may be life-threatening.

To date, the most effective method for treating hemophilia is to replenish the coagulation factor in which a patient is deficient, i.e., replacement therapy, which includes on-demand treatment and prophylactic treatment. On-demand treatment refers to a replacement therapy given during a bleeding episode of a patient to stop bleeding promptly. Prophylactic treatment refers to a regular replacement therapy given on a regular basis to prevent recurrent bleeding that can lead to joint deformities or life-threatening bleeding in a patient. The World Federation of Hemophilia Guidelines for the Management of Hemophilia (2020) strongly recommend that hemophilia patients with frequent and/or severe bleeding receive adequate prophylactic treatment and avoid bleeding as much as possible at any time. Even in countries with limited medical resources, prophylactic treatment is advocated over on-demand treatment; low-intensity prophylactic treatment is also acceptable. All types of prophylactic treatment are more beneficial than on-demand treatment. Early initiation of prophylactic treatment, i.e., primary prophylactic treatment, is recommended.

Some hemophilia patients develop coagulation factor inhibitors. Particularly, in patients with severe disease who have a long course of disease and have repeatedly used coagulation factor replacement therapy, the presence of inhibitors complicates treatment: 1. During bleeding episodes, high doses of FVIII/FIX agents or bypassing agents are required. Available bypassing agents include recombinant factor VIIa (rFVIIa) and prothrombin complex concentrate (PCC). As PCC may increase inhibitor reactivity, rFVIIa is the preferred choice of treatment, though its high cost poses a challenge to medication. 2. Conventional prophylactic treatment is difficult to carry out, especially for hemophilia B patients: In China, no drug has been approved for indications for prophylactic treatment of hemophilia B with inhibitor (HBW). Although there are drugs approved for prophylactic treatment of hemophilia A with inhibitor (HAW), their high costs limit widespread and long-term use.

Prophylactic treatment for patients with hemophilia with inhibitor remains an urgent clinical problem. Inhibiting the activity of tissue factor pathway inhibitor (TFPI) to promote thrombin generation may provide a new option for prophylactic treatment.

Tissue factor pathway inhibitor (TFPI) is a serine protease inhibitor that exists mainly in two molecular forms: TFPI-α and TFPI-β. TFPI-α consists of a negatively charged acidic N-terminus, three tandem repeated Kunitz-type protease inhibitory domains (K1, K2, and K3), and a positively charged basic C-terminus. TFPI-β contains K1, K2, and a C-terminus for glycosylphosphatidylinositol anchoring. TFPI is an important negative regulator in the extrinsic coagulation pathway: K2 binds to and inactivates activated coagulation factor X (FXa); subsequently, K1 rapidly binds to the adjacent tissue factor (TF)-activated coagulation factor FVII (FVIIa) complex in an FXa-dependent manner to form an FXa-TFPI-TF-FVIIa quaternary complex, inhibiting the catalytic activity of the TF-FVIIa complex and further preventing the activation of FX; in addition, the K3 domain can bind to protein S and immobilize it on the cell surface to improve the inhibitory activity against FXa. Inhibiting the activity of TFPI can eliminate its inhibitory effect on the activity of FXa and the TF-FVIIa complex, thereby sustaining thrombin generation and ameliorating the coagulation dysfunction in hemophilia patients.

### SUMMARY

### Method for Treating Hemophilia

In a first aspect, the present application relates to a method for treating hemophilia, comprising a step of administering to a subject in need thereof an antibody against tissue factor pathway inhibitor (TFPI) or an antigen-binding fragment thereof, wherein the antibody comprises a heavy chain variable region and a light chain variable region;
the heavy chain variable region comprises: a VH CDR1 comprising the amino acid sequence set forth in SEQ ID NO: 2, a VH CDR2 comprising the amino acid sequence set forth in SEQ ID NO: 3, and a VH CDR3 comprising the amino acid sequence set forth in SEQ ID NO: 4;
the light chain variable region comprises: a VL CDR1 comprising the amino acid sequence set forth in SEQ ID NO: 6, a VL CDR2 comprising the amino acid sequence set forth in SEQ ID NO: 7, and a VL CDR3 comprising the amino acid sequence set forth in SEQ ID NO: 8.

In some embodiments, the heavy chain variable region comprises the amino acid sequence set forth in SEQ ID NO: 1, or an amino acid sequence having at least 80%, at least 85%, at least 90%, at least 95%, or at least 99% sequence identity to SEQ ID NO: 1; for example, the heavy chain variable region may comprise an amino acid sequence having about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, or about 99% sequence identity to SEQ ID NO: 1.

In some embodiments, the light chain variable region comprises the amino acid sequence set forth in SEQ ID NO: 5, or an amino acid sequence having at least 80%, at least 85%, at least 90%, at least 95%, or at least 99% sequence identity to SEQ ID NO: 5; for example, the heavy chain variable region may comprise an amino acid sequence having about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, or about 99% sequence identity to SEQ ID NO: 5.

In some embodiments, the antibody comprises a heavy chain that further comprises a constant region of human IgG4 or a variant thereof, e.g., the amino acid sequence set forth in SEQ ID NO: 9.

In some embodiments, the antibody comprises a light chain that further comprises a constant region of human Igκ or a variant thereof, e.g., the amino acid sequence set forth in SEQ ID NO: 10.

In some embodiments, the antibody comprises a heavy chain that comprises the amino acid sequence set forth in SEQ ID NO: 11.

In some embodiments, the antibody comprises a light chain that comprises the amino acid sequence set forth in SEQ ID NO: 12.

In some embodiments, the treating is prophylactic treatment.

In some embodiments, the hemophilia is hemophilia A; for example, the hemophilia may be hemophilia A with inhibitor or hemophilia A without inhibitor. In some embodiments, the hemophilia A is severe and moderate-to-severe hemophilia A (factor VIII activity level ≤ 2%).

In some embodiments, the hemophilia is hemophilia B; for example, the hemophilia may be hemophilia B with inhibitor or hemophilia B without inhibitor. In some embodiments, the hemophilia B is severe and moderate-to-severe hemophilia B (factor IX activity level ≤ 2%).

In some embodiments, the hemophilia is hemophilia A with or without inhibitor or hemophilia B with or without inhibitor.

In some embodiments, the hemophilia is von Willebrand disease.

In some embodiments, the hemophilia is von Willebrand disease type 1, type 2, or type 3.

In some embodiments, the subject is a male hemophilia patient aged 12 years-70 years.

In some embodiments, the subject has a body weight of ≥30 kg and a body mass index (BMI) of <28 kg/m².

In some embodiments, the subject is a patient with hemophilia A; for example, the subject may be a patient with severe and moderate-to-severe hemophilia A (factor VIII activity level ≤ 2%).

In some embodiments, the subject is a patient with hemophilia A with inhibitor or a patient with hemophilia A without inhibitor.

In some embodiments, the subject is a patient with hemophilia B; for example, the subject may be a patient with severe and moderate-to-severe hemophilia B (factor IX activity level ≤ 2%).

In some embodiments, the subject is a patient with hemophilia B with inhibitor or a patient with hemophilia B without inhibitor.

In some embodiments, the subject is a patient with von Willebrand disease; for example, the subject may be a patient with von Willebrand disease type 1, type 2, or type 3.

In some embodiments, the subject has not previously used a TFPI antibody-based drug.

In some embodiments, the treatment method comprises administering the antibody or the antigen-binding fragment thereof once weekly at a dose of 1.0 mg/kg-2.0 mg/kg, e.g., 1.0 mg/kg, 1.05 mg/kg, 1.1 mg/kg, 1.15 mg/kg, 1.2 mg/kg, 1.25 mg/kg, 1.3 mg/kg, 1.35 mg/kg, 1.4 mg/kg, 1.45 mg/kg, 1.5 mg/kg, 1.55 mg/kg, 1.6 mg/kg, 1.65 mg/kg, 1.7 mg/kg, 1.75 mg/kg, 1.8 mg/kg, 1.85 mg/kg, 1.9 mg/kg, 1.95 mg/kg, or 2.0 mg/kg, as calculated based on the subject's body weight.

In some embodiments, the subject has a body weight of ≥30 kg.

In some embodiments, the subject has a body weight of ≥50 kg; or, the subject has a body weight of ≥40 kg and <50 kg; or, the subject has a body weight of ≥30 kg and <40 kg.

In some embodiments, the treatment method comprises administering the antibody or the antigen-binding fragment thereof once weekly at a dose of 1.5 mg/kg, as calculated based on the subject's body weight.

In some embodiments, the treatment method comprises administering the antibody or the antigen-binding fragment thereof once weekly at a dose of 1.5 mg/kg, as calculated based on the subject's body weight; the subject has a body weight of ≥50 kg.

In some embodiments, the treatment method comprises administering the antibody or the antigen-binding fragment thereof once weekly at a dose of 1.75 mg/kg, as calculated based on the subject's body weight.

In some embodiments, the treatment method comprises administering the antibody or the antigen-binding fragment thereof once weekly at a dose of 1.75 mg/kg, as calculated based on the subject's body weight; the subject has a body weight of ≥40 kg and <50 kg.

In some embodiments, the treatment method comprises administering the antibody or the antigen-binding fragment thereof once weekly at a dose of 1.5 mg/kg-1.75 mg/kg, as calculated based on the subject's body weight.

In some embodiments, the treatment method comprises administering the antibody or the antigen-binding fragment thereof once weekly at a dose of 1.5 mg/kg-1.75 mg/kg, as calculated based on the subject's body weight; the subject has a body weight of ≥40 kg.

In some embodiments, the treatment method comprises administering the antibody or the antigen-binding fragment thereof once weekly at a dose of 2.0 mg/kg, as calculated based on the subject's body weight.

In some embodiments, the treatment method comprises administering the antibody or the antigen-binding fragment thereof once weekly at a dose of 2.0 mg/kg, as calculated based on the subject's body weight; the subject has a body weight of ≥30 kg and <40 kg.

In some embodiments, the treatment method comprises administering the antibody or the antigen-binding fragment thereof once weekly at a dose of 1.5 mg/kg-2.0 mg/kg, as calculated based on the subject's body weight.

In some embodiments, the treatment method comprises administering the antibody or the antigen-binding fragment thereof once weekly at a dose of 1.5 mg/kg-2.0 mg/kg, as calculated based on the subject's body weight; the subject has a body weight of ≥30 kg.

In some embodiments, the antibody or the antigen-binding fragment thereof may be administered by intravenous drip infusion or subcutaneous injection; preferably, the antibody or the antigen-binding fragment thereof is administered by subcutaneous injection.

In some embodiments, the treatment method comprises subcutaneously injecting the antibody or the antigen-binding fragment thereof once weekly at a dose of 1.0 mg/kg, 1.5 mg/kg, or 2.0 mg/kg, as calculated based on the subject's body weight. In some embodiments, the treatment method comprises subcutaneously injecting the antibody or the antigen-binding fragment thereof once weekly at a dose of 1.5 mg/kg-2.0 mg/kg, as calculated based on the subject's body weight.

In some embodiments, the treatment method comprises subcutaneously injecting the antibody or the antigen-binding fragment thereof once weekly at a dose of 1.5 mg/kg-2.0 mg/kg, as calculated based on the subject's body weight; the subject has a body weight of ≥30 kg.

In some embodiments, the treatment method comprises subcutaneously injecting the antibody or the antigen-binding fragment thereof once weekly at a dose of 1.5 mg/kg, as calculated based on the subject's body weight; the subject has a body weight of ≥50 kg.

In some embodiments, the treatment method comprises subcutaneously injecting the antibody or the antigen-binding fragment thereof once weekly at a dose of 1.75 mg/kg, as calculated based on the subject's body weight; the subject has a body weight of ≥40 kg and <50 kg.

In some embodiments, the treatment method comprises subcutaneously injecting the antibody or the antigen-binding fragment thereof once weekly at a dose of 2.0 mg/kg, as calculated based on the subject's body weight; the subject has a body weight of ≥30 kg and <40 kg.

In some embodiments, after the antibody or the antigen-binding fragment is administered, the subject has an annualized bleeding rate (ABR) of no more than 10.0, preferably no more than 8.0, more preferably no more than 5.0, and even more preferably no more than 2.0. For example, the subject has an annualized bleeding rate of no more than 9.5, no more than 9.0, no more than 8.5, no more than 8.0, no more than 7.5, no more than 7.0, no more than 6.5, no more than 6.0, no more than 5.5, no more than 5.0, no more than 4.5, no more than 4.0, no more than 3.5, no more than 3.0, no more than 2.5, no more than 2.0, no more than 1.5, or no more than 1.0.

In some embodiments, after the antibody or the antigen-binding fragment is administered, the subject has an annualized bleeding rate that decreases by no less than 60%, preferably no less than 70%, more preferably no less than 80%, and even more preferably no less than 90%, compared to that before the administration. For example, the subject has an annualized bleeding rate that decreases by no less than 65%, no less than 70%, no less than 75%, no less than 80%, no less than 81%, no less than 82%, no less than 83%, no less than 84%, no less than 85%, no less than 86%, no less than 87%, no less than 88%, no less than 89%, no less than 90%, no less than 91%, no less than 92%, no less than 93%, no less than 94%, no less than 95%, no less than 96%, no less than 97%, no less than 98%, or no less than 99% compared to that before the administration.

The annualized bleeding rate (ABR) is calculated as: ABR = the number of bleeding episodes within follow-up time (days) ÷ the number of follow-up days × 365.25.

In some embodiments, the annualized bleeding rate is calculated based on spontaneous and traumatic bleeding.

### Use

In a second aspect, the present application further relates to an antibody against tissue factor pathway inhibitor (TFPI) or an antigen-binding fragment thereof for treating hemophilia, wherein the antibody comprises a heavy chain variable region and a light chain variable region;
the heavy chain variable region comprises: a VH CDR1 comprising the amino acid sequence set forth in SEQ ID NO: 2, a VH CDR2 comprising the amino acid sequence set forth in SEQ ID NO: 3, and a VH CDR3 comprising the amino acid sequence set forth in SEQ ID NO: 4;
the light chain variable region comprises: a VL CDR1 comprising the amino acid sequence set forth in SEQ ID NO: 6, a VL CDR2 comprising the amino acid sequence set forth in SEQ ID NO: 7, and a VL CDR3 comprising the amino acid sequence set forth in SEQ ID NO: 8.

In a third aspect, the present application further relates to use of an antibody against tissue factor pathway inhibitor (TFPI) or an antigen-binding fragment thereof in the preparation of a medicament for treating hemophilia, wherein the antibody comprises a heavy chain variable region and a light chain variable region;
the heavy chain variable region comprises: a VH CDR1 comprising the amino acid sequence set forth in SEQ ID NO: 2, a VH CDR2 comprising the amino acid sequence set forth in SEQ ID NO: 3, and a VH CDR3 comprising the amino acid sequence set forth in SEQ ID NO: 4;
the light chain variable region comprises: a VL CDR1 comprising the amino acid sequence set forth in SEQ ID NO: 6, a VL CDR2 comprising the amino acid sequence set forth in SEQ ID NO: 7, and a VL CDR3 comprising the amino acid sequence set forth in SEQ ID NO: 8.

The second aspect and the third aspect are further defined as follows:
In some embodiments, the heavy chain variable region comprises the amino acid sequence set forth in SEQ ID NO: 1, or an amino acid sequence having at least 80%, at least 85%, at least 90%, at least 95%, or at least 99% sequence identity to SEQ ID NO: 1; for example, the heavy chain variable region may comprise an amino acid sequence having about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, or about 99% sequence identity to SEQ ID NO: 1.

In some embodiments, the light chain variable region comprises the amino acid sequence set forth in SEQ ID NO: 5, or an amino acid sequence having at least 80%, at least 85%, at least 90%, at least 95%, or at least 99% sequence identity to SEQ ID NO: 5; for example, the heavy chain variable region may comprise an amino acid sequence having about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, or about 99% sequence identity to SEQ ID NO: 5.

In some embodiments, the antibody comprises a heavy chain that further comprises a constant region of human IgG4 or a variant thereof, e.g., the amino acid sequence set forth in SEQ ID NO: 9.

In some embodiments, the antibody comprises a light chain that further comprises a constant region of human Igκ or a variant thereof, e.g., the amino acid sequence set forth in SEQ ID NO: 10.

In some embodiments, the antibody comprises a heavy chain that comprises the amino acid sequence set forth in SEQ ID NO: 11.

In some embodiments, the antibody comprises a light chain that comprises the amino acid sequence set forth in SEQ ID NO: 12.

In some embodiments, the treating is prophylactic treatment.

In some embodiments, the hemophilia is hemophilia A; for example, the hemophilia may be hemophilia A with inhibitor or hemophilia A without inhibitor. In some embodiments, the hemophilia A is severe and moderate-to-severe hemophilia A (factor VIII activity level ≤ 2%).

In some embodiments, the hemophilia is hemophilia B; for example, the hemophilia may be hemophilia B with inhibitor or hemophilia B without inhibitor. In some embodiments, the hemophilia B is severe and moderate-to-severe hemophilia B (factor IX activity level ≤ 2%).

In some embodiments, the hemophilia is hemophilia A with or without inhibitor or hemophilia B with or without inhibitor.

In some embodiments, the hemophilia is von Willebrand disease.

In some embodiments, the hemophilia is von Willebrand disease type 1, type 2, or type 3.

In some embodiments, the antibody or the antigen-binding fragment thereof is administered once weekly at a dose of 1.0 mg/kg-2.0 mg/kg, e.g., 1.0 mg/kg, 1.05 mg/kg, 1.1 mg/kg, 1.15 mg/kg, 1.2 mg/kg, 1.25 mg/kg, 1.3 mg/kg, 1.35 mg/kg, 1.4 mg/kg, 1.45 mg/kg, 1.5 mg/kg, 1.55 mg/kg, 1.6 mg/kg, 1.65 mg/kg, 1.7 mg/kg, 1.75 mg/kg, 1.8 mg/kg, 1.85 mg/kg, 1.9 mg/kg, 1.95 mg/kg, or 2.0 mg/kg, as calculated based on the subject's body weight.

In some embodiments, the subject has a body weight of ≥30 kg.

In some embodiments, the subject has a body weight of ≥50 kg; or, the subject has a body weight of ≥40 kg and <50 kg; or, the subject has a body weight of ≥30 kg and <40 kg.

In some embodiments, the antibody or the antigen-binding fragment thereof is administered once weekly at a dose of 1.5 mg/kg, as calculated based on the subject's body weight.

In some embodiments, the antibody or the antigen-binding fragment thereof is administered once weekly at a dose of 1.5 mg/kg, as calculated based on the subject's body weight; the subject has a body weight of ≥50 kg.

In some embodiments, the antibody or the antigen-binding fragment thereof is administered once weekly at a dose of 1.75 mg/kg, as calculated based on the subject's body weight.

In some embodiments, the antibody or the antigen-binding fragment thereof is administered once weekly at a dose of 1.75 mg/kg, as calculated based on the subject's body weight; the subject has a body weight of ≥40 kg and <50 kg.

In some embodiments, the antibody or the antigen-binding fragment thereof is administered once weekly at a dose of 1.5 mg/kg-1.75 mg/kg, as calculated based on the subject's body weight.

In some embodiments, the antibody or the antigen-binding fragment thereof is administered once weekly at a dose of 1.5 mg/kg-1.75 mg/kg, as calculated based on the subject's body weight; the subject has a body weight of ≥40 kg.

In some embodiments, the antibody or the antigen-binding fragment thereof is administered once weekly at a dose of 2.0 mg/kg, as calculated based on the subject's body weight.

In some embodiments, the antibody or the antigen-binding fragment thereof is administered once weekly at a dose of 2.0 mg/kg, as calculated based on the subject's body weight; the subject has a body weight of ≥30 kg and <40 kg.

In some embodiments, the antibody or the antigen-binding fragment thereof is administered once weekly at a dose of 1.5 mg/kg-2.0 mg/kg, as calculated based on the subject's body weight.

In some embodiments, the antibody or the antigen-binding fragment thereof is administered once weekly at a dose of 1.5 mg/kg-2.0 mg/kg, as calculated based on the subject's body weight; the subject has a body weight of ≥30 kg.

In some embodiments, the antibody or the antigen-binding fragment thereof may be administered by intravenous drip infusion or subcutaneous injection; preferably, the antibody or the antigen-binding fragment thereof is administered by subcutaneous injection.

In some embodiments, the antibody or the antigen-binding fragment thereof is injected subcutaneously once weekly at a dose of 1.0 mg/kg, 1.5 mg/kg, or 2.0 mg/kg, as calculated based on the subject's body weight.

In some embodiments, the antibody or the antigen-binding fragment thereof is injected subcutaneously once weekly at a dose of 1.5 mg/kg-2.0 mg/kg, as calculated based on the subject's body weight.

In some embodiments, the antibody or the antigen-binding fragment thereof is injected subcutaneously once weekly at a dose of 1.5 mg/kg-2.0 mg/kg, as calculated based on the subject's body weight; the subject has a body weight of ≥30 kg.

In some embodiments, the antibody or the antigen-binding fragment thereof is injected subcutaneously once weekly at a dose of 1.5 mg/kg, as calculated based on the subject's body weight; the subject has a body weight of ≥50 kg.

In some embodiments, the antibody or the antigen-binding fragment thereof is injected subcutaneously once weekly at a dose of 1.75 mg/kg, as calculated based on the subject's body weight; the subject has a body weight of ≥40 kg and <50 kg. In some embodiments, the antibody or the antigen-binding fragment thereof is injected subcutaneously once weekly at a dose of 2.0 mg/kg, as calculated based on the subject's body weight; the subject has a body weight of ≥30 kg and <40 kg. In some embodiments, after the antibody or the antigen-binding fragment is administered, the subject has an annualized bleeding rate (ABR) of no more than 10.0, preferably no more than 8.0, more preferably no more than 5.0, and even more preferably no more than 2.0. For example, the subject has an annualized bleeding rate of no more than 9.5, no more than 9.0, no more than 8.5, no more than 8.0, no more than 7.5, no more than 7.0, no more than 6.5, no more than 6.0, no more than 5.5, no more than 5.0, no more than 4.5, no more than 4.0, no more than 3.5, no more than 3.0, no more than 2.5, no more than 2.0, no more than 1.5, or no more than 1.0.

In some embodiments, after the antibody or the antigen-binding fragment is administered, the subject has an annualized bleeding rate that decreases by no less than 60%, preferably no less than 70%, more preferably no less than 80%, and even more preferably no less than 90%, compared to that before the administration. For example, the subject has an annualized bleeding rate that decreases by no less than 65%, no less than 70%, no less than 75%, no less than 80%, no less than 81%, no less than 82%, no less than 83%, no less than 84%, no less than 85%, no less than 86%, no less than 87%, no less than 88%, no less than 89%, no less than 90%, no less than 91%, no less than 92%, no less than 93%, no less than 94%, no less than 95%, no less than 96%, no less than 97%, no less than 98%, or no less than 99% compared to that before the administration.

### Definitions of Terms

In the present disclosure, unless otherwise specified, the scientific and technical nouns used herein have the meanings generally understood by those skilled in the art. In addition, the terms and laboratory procedures related to protein and nucleic acid chemistry, molecular biology, cell and tissue culture, microbiology, and immunology used herein are all terms and conventional procedures widely used in the corresponding fields. Moreover, in order to facilitate a better understanding of the present disclosure, the definitions and explanations of related terms are provided below.

As used herein, the term "tissue factor pathway inhibitor" or "TFPI" refers to any variants, isotypes, and species homologs of human TFPI that are naturally expressed by cells. Illustratively, human TFPI comprises the amino acid sequence set forth in SEQ ID NO: 13.

The term "antibody" refers to immunoglobulins and immunoglobulin fragments, whether naturally or partially or completely synthetically (e.g., recombinantly) produced, including any fragment thereof containing at least a portion of the variable region of the immunoglobulin molecule that retains the binding specificity ability of the full-length immunoglobulin. Hence, an antibody includes any protein that has a binding domain that is homologous or substantially homologous to an immunoglobulin antigen-binding domain (antibody binding site). Antibodies include antibody fragments, such as anti-tumor cell antibody fragments. As used herein, the term antibody, thus, includes synthetic antibodies, recombinantly produced antibodies, multispecific antibodies (e.g., bispecific antibodies), human antibodies, non-human antibodies, humanized antibodies, chimeric antibodies, intracellular antibodies, and antibody fragments, such as, but not limited to, Fab fragments, Fab' fragments, F(ab')₂ fragments, Fv fragments, disulfide bond-linked Fvs (dsFvs), Fd fragments, Fd' fragments, single-chain Fvs (scFvs), single-chain Fabs (scFabs), diabodies, anti-idiotypic (anti-Id) antibodies, or antigen-binding fragments of any of the above antibodies. Antibodies provided herein include members of any immunoglobulin type (e.g., IgG, IgM, IgD, IgE, IgA, and IgY), any class (e.g., IgG1, IgG2, IgG3, IgG4, IgA1, and IgA2), or subclass (e.g., IgG2a and IgG2b).

A full-length antibody generally refers to an immunoglobulin molecule consisting of two identical pairs of polypeptide chains (each pair has one "light" (L) chain and one "heavy" (H) chain). Antibody light chains can be classified as κ and λ light chains. Heavy chains can be classified as µ, δ, γ, α, or ε, and the isotypes of antibodies are defined as IgM, IgD, IgG, IgA, and IgE, respectively. Within a light chain and a heavy chain, the variable regions and the constant regions can be linked by a "J" region of about 12 or more amino acids. The heavy chain can further comprise a "D" region of about 3 or more amino acids. The heavy chain may consist of a heavy chain variable region (VH) and a heavy chain constant region (CH). The heavy chain constant region may consist of 3 domains (CH1, CH2, and CH3). The light chain may consist of a light chain variable region (VL) and a light chain constant region (CL). The light chain constant region may consist of one domain: CL. The constant regions of an antibody can mediate the binding of immunoglobulins to host tissues or factors, including various cells of the immune system (e.g., effector cells) and the first component of the classical complement system (C1q). The VH and VL regions can also be subdivided into regions with high variability (known as complementarity determining regions (CDRs)) interspersed with relatively conserved regions known as framework regions (FRs). For example, the VH and VL may each comprise or consist of 3 CDRs and 4 FRs arranged from the amino-terminus to the carboxy-terminus in the following order: FR1, CDR1, FR2, CDR2, FR3, CDR3, FR4. The variable regions (VH and VL) of each heavy/light chain pair form an antibody binding site.

The term "antibody fragment" or "antigen-binding fragment" refers to any portion of a full-length antibody that is less than full length but contains at least a portion of the variable region of the antibody that binds to an antigen (e.g., one or more CDRs and/or one or more antigen-binding sites) and thus retains the binding specificity and at least a portion of the specific binding ability of the full-length antibody. Hence, an antigen-binding fragment refers to an antibody fragment that contains an antigen-binding portion that binds to the same antigen as the antibody from which the antibody fragment is derived. Antibody fragments include antibody derivatives produced by enzymatic treatment of full-length antibodies, as well as synthetically produced derivatives, e.g., recombinantly produced derivatives. Antibodies include antibody fragments. Examples of antibody fragments include, but are not limited to, Fab, Fab', F(ab')2, single-chain Fv (scFv), Fv, dsFv, diabodies, Fd and Fd' fragments, and other fragments, including modified fragments (see, e.g., Methods in Molecular Biology, Vol 207: Recombinant Antibodies for Cancer Therapy Methods and Protocols (2003); Chapter 1; p 3-25, Kipriyanov). The fragment may include multiple chains linked together, e.g., by disulfide bonds and/or by peptide linkers. An antibody fragment generally contains at least or about 50 amino acids and typically at least or about 200 amino acids. An antigen-binding fragment includes any antibody fragment that, when inserted into an antibody framework (e.g., by replacing a corresponding region), results in an antibody that immunospecifically binds (i.e., exhibits a Ka of at least or at least about 10⁷-10⁸M⁻¹) to the antigen.

"Complementarity determining regions" or "CDR regions" or "CDRs" are regions in an antibody variable domain that are highly variable in sequence and form structurally defined loops ("hypervariable loops") and/or comprise antigen contact residues ("antigen contact points"). CDRs are primarily responsible for binding to antigenic epitopes. The CDRs of heavy and light chains are generally referred to as CDR1, CDR2, and CDR3, and are sequentially numbered from the N-terminus. The CDRs located in a heavy chain variable domain of an antibody are referred to as HCDR1, HCDR2, and HCDR3, and the CDRs located in a light chain variable domain of an antibody are referred to as LCDR1, LCDR2, and LCDR3. In the amino acid sequence of a given light or heavy chain variable region, the exact amino acid sequence boundaries of the CDRs can be determined using any one or a combination of many well-known antibody CDR assignment systems, including, e.g., Chothia, which is based on the three-dimensional structures of antibodies and the topology of CDR loops (Chothia et al., (1989) Nature 342: 877-883; Al-Lazikani et al., "Standard conformations for the canonical structures of immunoglobulins", Journal of Molecular Biology, 273: 927-948 (1997)), Kabat, which is based on antibody sequence variability (Kabat et al., Sequences of Proteins of Immunological Interest, 4th Edition, U.S. Department of Health and Human Services, National Institutes of Health (1987)), AbM (University of Bath), Contact (University College London), International ImMunoGeneTics database (IMGT) (imgt.cines.fr/ on the World Wide Web), and the North CDR definition, which is based on an affinity propagation clustering using a large number of crystal structures (North et al., "A New Clustering of Antibody CDR Loop Conformations", Journal of Molecular Biology, 406, 228-256 (2011)).

The regional ranges of the CDRs defined using the Kabat, AbM, IMGT, or Chothia scheme are as follows:

| CDR | Kabat scheme | AbM scheme | IMGT scheme | Chothia scheme |
|---|---|---|---|---|
| LCDR1 (Kabat and Chothia numbering systems) | L24-L34 | L24-L34 | L27-L32 | L26-L32 |
| LCDR2 (Kabat and Chothia numbering systems) | L50-L56 | L50-L56 | L50-L52 | L50-L52 |
| LCDR3 (Kabat and Chothia numbering systems) | L89-L97 | L89-L97 | L89-L96 | L91-L96 |
| HCDR1 (Kabat numbering system) | H31-H35B | H26-H35B | H26-H35B | H26-H32 |
| HCDR1 (Chothia numbering system) | H31-H35 | H26-H35 | H26-H35 | H26-H32 |
| HCDR2 (Kabat and Chothia numbering systems) | H50-H65 | H50-H58 | H51-H57 | H53-H55 |
| HCDR3 (Kabat and Chothia numbering systems) | H95-H102 | H95-H102 | H93-H102 | H96-H101 |

Unless otherwise specified, the CDR regions are defined using the Kabat scheme in the present application.

As used herein, the term "and/or" refers to any one of the options or two or more of the options.

As used herein, the term "comprise", "contain", or "include" refers to including the stated elements, integers, or steps, but not excluding any other elements, integers, or steps. As used herein, the term "comprise", "contain", or "include" also encompasses combinations of the stated elements, integers, or steps, unless otherwise indicated.

The term "administer" generally refers to a method of giving a subject (e.g., a patient) a certain dose of a compound or pharmaceutical composition. Administration may be performed by any suitable means, including parenteral, intrapulmonary, and intranasal, as well as (if topical treatment is desired) intralesional administration. Parenteral infusion includes, for example, intramuscular, intravenous, intraarterial, intraperitoneal, or subcutaneous administration.

In the present application, the term "about" generally means varying by 0.5%-10% above or below the stated value, for example, varying by 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5% or 10% above or below the stated value. Unless otherwise specified, all numerical values mentioned in the present application are seen to be modified by "about". If there is a question or there is no common understanding for the margin of error of a particular value or parameter acknowledged in the art, "about" means ±5% of that value or parameter.

The term "effective amount" refers to an amount or dose of the antibody or fragment or composition or combination of the present disclosure that produces the expected effects in a patient in need of treatment or prevention after being administered to the patient in a single dose or multiple doses. "Therapeutically effective amount" and "prophylactically effective amount" may be included according to the expected effects.

"Therapeutically effective amount" refers to an amount effective, at dosages and for periods of time necessary, to achieve the desired therapeutic result. A therapeutically effective amount is also one in which any toxic or detrimental effect of the antibody or antibody fragment or composition or combination is outweighed by the therapeutically beneficial effects. "Prophylactically effective amount" refers to an amount effective, at dosages and for periods of time necessary, to achieve the desired prophylactic result. In general, as a prophylactic dose is used in a subject prior to or at an earlier stage of a disease, the prophylactically effective amount will be less than the therapeutically effective amount.

"Individual" or "subject" includes mammals. Mammals include, but are not limited to, domestic animals (e.g., cows, sheep, cats, dogs, and horses), primates (e.g., human and non-human primates such as monkeys), rabbits, and rodents (e.g., mice and rats). In some embodiments, the individual or subject is a human.

### BRIEF DESCRIPTION OF THE DRAWINGS

The drawings are briefly described below:
FIG. 1 shows the mean dPT results after *in vitro* treatment of plasma from patients with different types of hemophilia and VWD3 patients with different concentrations of KN057.
FIGs. 2A-2E show a comparison of the dPT results after *in vitro* treatment of plasma from hemophilia patients and VWD3 patients with different concentrations of KN057 and PF-06741086.
FIGs. 3A and 3B show the TGA test results after *in vitro* treatment of plasma from patients with different types of hemophilia and VWD3 patients with different concentrations of KN057.
FIGs. 4A-4E show a comparison of the peak thrombin results after *in vitro* treatment of plasma from hemophilia patients and VWD3 patients with different concentrations of KN057 and PF-06741086.
FIGs. 5A-5E show a comparison of the ETP results after *in vitro* treatment of plasma from hemophilia patients and VWD3 patients with different concentrations of KN057 and PF-06741086.

### DETAILED DESCRIPTION

### List of abbreviations

| English abbreviation | Full name in English |
|---|---|
| FVIII | Factor VIII |
| FIX | Factor IX |
| FX | Factor X |
| FXa | Activated Coagulation Factor X |
| TF | Tissue Factor |
| FVIIa | Activated Coagulation Factor VII |
| rFVIIa | Recombinant Factor VIIa |
| PCC | Prothrombin Complex Concentrate |
| TFPI | Tissue Factor Pathway Inhibitor |
| HA | Hemophilia A without Inhibitor |
| HB | Hemophilia B without Inhibitor |
| HAW | Hemophilia A with Inhibitor |
| HBW | Hemophilia B with Inhibitor |
| VWD3 | Von Willebrand Disease Type 3 |
| ABR | Annualized bleeding rate |
| PK | Pharmacokinetics |
| PD | Pharmacodynamics |
| dPT | diluted prothrombin time |
| TGA | thrombin generation assay |
| ETP | endogenous thrombin potential |
| ADA | Anti-drug Antibody |
| Nab | Neutralizing Antibody |
| Tₘₐₓ | Time to maximum plasma concentration |
| Cₘₐₓ | Maximum Concentration |
| AUCₗₐₛₜ | Area under the curve time zero to time of last measurable concentration |
| CL | Plasma Clearance |
| t_{1/2} | Time Half |
| AE | Adverse Event |
| SAE | Serious Adverse Event |
| TEAE | Treatment Emergent Adverse Event |
| CTCAE | Common Terminology Criteria for Adverse Events |
| HJHS | Hemophilia Joint Health Score |
| EQ-5D-5L | EuroQol Five Dimensions Five Level Questionnaire |
| Free TFPI | Free Tissue Factor Pathway Inhibitor |
| PF1+2 | Prothrombin Fragment 1+2 |
| ECG | Electrocardiogram |

### Example 1. Preparation of Anti-TFPI Antibodies

KN057 is a humanized anti-TFPI monoclonal antibody. The amino acid sequence of its heavy chain is set forth in SEQ ID NO: 11, and the amino acid sequence of its light chain is set forth in SEQ ID NO: 12. It can be prepared by referring to the method described in Patent Application WO2021037197.

The amino acid sequences of PF-06741086 (marstacimab) are published in WHO Drug Information, Vol. 32, No. 2, 2018; INN Proposed List 119. Vectors containing nucleic acid sequences capable of encoding the above antibody were transfected into cells, and after expression and purification, the corresponding antibody was obtained.

### Example 2. Phase II Clinical Study of KN057 in Treatment of Hemophilia

### • Trial objective:

To evaluate the safety, tolerability, pharmacokinetics, pharmacodynamic profile, immunogenicity, and effectiveness of multiple subcutaneous injections of KN057 in patients with HA or HB with or without inhibitor.

### • Administration regimen

Subjects will receive one subcutaneous injection of a KN057 injectable solution every week. The study includes a total of 3 dose groups: 1.0 mg/kg, 1.5 mg/kg, and 2.0 mg/kg.

### • Subject inclusion criteria

1. Male, aged 18-70 years (inclusive), body weight ≥ 40 kg.
2. Severe and moderate-to-severe HA or HB (factor VIII activity or factor IX activity level ≤ 2%).
3. Patients without inhibitors must also meet the following criteria: a) negative for FVIII or FIX inhibitors during the screening period; b) ≥6 treatment-requiring bleeding episodes (spontaneous and/or traumatic, excluding surgical bleeding) within the last 6 months, and willing to use the study drug in combination with on-demand treatment during the study; and c) no fewer than 50 exposure days of coagulation factor replacement therapy prior to screening.
4. Patients with inhibitors must also meet the following criteria: a) positive for FVIII or FIX inhibitors during the screening period; and b) ≥6 treatment-requiring bleeding episodes (spontaneous and/or traumatic, excluding surgical bleeding) within the last 6 months, and willing to use the study drug in combination with on-demand treatment during the study.
5. Able and willing to undergo washout periods for the original treatment regimen before receiving KN057: a washout period of at least 48 hours for recombinant factor VIIa (rFVIIa), a washout period of at least 72 hours for FVIII and prothrombin complex concentrate (PCC), a washout period of at least 96 hours for FIX, and a washout period of at least 5 drug half-lives for other drugs or clinical trial drugs that have relatively long half-lives, such as emicizumab.
6. Willing to accept the trial, able to comply with the relevant management regulations of the institute that carries out the study, and able to complete administration, follow-ups, and protocol-required examinations during the study.

### • Number of subjects

The target number of subjects enrolled is 24.

### • Endpoints

### Primary endpoints:

1) The incidence and severity of TEAEs (D85).
2) The number of patients withdrawn from the study due to TEAEs (D85).
3) The frequency and severity of abnormalities in various laboratory safety indicators (D85).
4) Changes in safety indicators in ECGs, vital signs, and physical examinations (D85).
5) Injection site reactions (D85).

### Secondary endpoints:

1) After the first dose: maximum plasma concentration (Cmax), time to maximum plasma concentration (Tmax), and area under the post-dose plasma concentration-time curve (AUClast) (D8).
2) After administration on D36: maximum plasma concentration (Cmax, ss), time to Cmax, ss (Tmax, ss), area under the plasma concentration-time curve (AUCτ), apparent clearance (CL/F), and minimum plasma concentration (Cmin) (D43).
3) Pharmacodynamic indicators: total TFPI and free TFPI; thrombin generation assay (TGA); and prothrombin fragment 1+2 (PF1+2) concentration and changes from baseline (D85 and D169).
4) Immunogenicity: the number and proportion of patients developing anti-KN057 antibodies (ADAs); the number and proportion of patients developing anti-KN057 neutralizing antibodies (NAbs) (NAb detection will be performed only for ADA-positive samples and corresponding baseline samples) (D85, D169).
5) Effectiveness assessment: annualized bleeding rate (ABR) (D85, D169).

Note: In this example, D followed by number n denotes the nth day after the first dose of KN057.

### • Summary of trial results

The study enrolled 24 subjects, with 6 cases of HA in group 1 (1.0 mg/kg, once weekly), 6 cases of HA in group 2 (1.5 mg/kg, once weekly), 6 cases of HAW in group 4 (1.5 mg/kg, once weekly), and 5 cases of HA and 1 case of HB in group 3 (2.0 mg/kg, once weekly). Safety results: Among the adverse events related to the trial drug that the subjects experienced, only "fibrinogen decreased" reached grade 2 according to CTCAE 5.0, and the rest were all grade 1; no subjects experienced SAEs related to the trial drug. KN057 demonstrated good safety and tolerability at doses of 1.0 mg/kg, 1.5 mg/kg, and 2.0 mg/kg in subjects.

Efficacy results: The annualized bleeding rate (ABR) of the subjects in the 1.0 mg/kg dose group (group 1) had decreased, but the efficacy had not met the expectations. The ABRs of the 1.5 mg/kg dose groups during the main trial period (a 12-week treatment period) significantly decreased: in group 2 (HA), the mean and median ABRs were 7.648 and 2.03, respectively, and further decreased to 5.083 and 1.05 during the full treatment period (a 24-week treatment period), and 3 out of 6 subjects (50%) had an ABR of 0 during the full treatment period; in group 4 (HAW), the mean and median ABRs during the main trial period were 1.255 and 0.00, respectively, the efficacy was sustained and the ABRs were comparable during the full treatment period, and 4 out of 6 subjects (66.7%) had an ABR of 0 during the full treatment period. The ABR of the 2.0 mg/kg dose group (group 3) also decreased relative to baseline but was higher than those of the 1.5 mg/kg dose groups, which was considered to be primarily due to the fact that the number of cases enrolled was small because it is a rare disease and to the relatively large differences in individual disease status and efficacy in the 2.0 mg/kg dose group.

One subject in group 2 experienced right hip pain several times, which was considered bleeding and was treated with FVIII. An imaging examination was subsequently performed, and the investigator determined that it was actually not true bleeding. Thus, a sensitivity analysis excluding this subject's ABR was performed, and the results showed that the mean and median ABRs of group 2 during the main trial period were 2.400 and 0.00, respectively, which were similar to the results for group 4.

By analyzing the PD indicators in the trial, it can be found that free TFPI was negatively correlated with drug concentration, and total TFPI and PF1+2 were positively correlated with drug concentration; the magnitude of changes in PD indicators (free TFPI, total TFPI, and PF1+2) increased with dose and showed a certain correlation with dose; the inhibition of change in free TFPI was not further enhanced as the dose was raised from 1.5 mg/kg to 2.0 mg/kg, and PF1+2 tended to increase in a dose-dependent manner at 2.0 mg/kg. According to the safety, effectiveness, and PD results, the Phase III dose of KN057 was determined to be 1.5 mg/kg, administered once weekly.

Note: The annualized bleeding rate during the main trial period = (the number of treatment-requiring bleeding episodes during the period from D1 to [D85+6/the date of early withdrawal, whichever is earlier, if the subject did not enter the D92 visit; or D92-1 if the subject entered the D92 visit]) ÷ (the date of [D85+6/the date of early withdrawal, whichever is earlier, if the subject did not enter the D92 visit; or D92-1 if the subject entered the D92 visit] - the date of the first dose + 1) × 365.25. 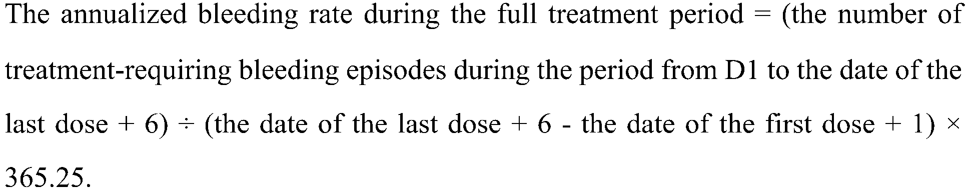 The annualized bleeding rate during the full treatment period = (the number of treatment-requiring bleeding episodes during the period from D1 to the date of the last dose + 6) ÷ (the date of the last dose + 6 - the date of the first dose + 1) × 365.25.

### Example 3. Randomized, Open-Label Study for Evaluating Effectiveness and Safety of Prophylactic Treatment with KN057 Injectable Solution in Patients with Hemophilia A and Hemophilia B with Inhibitor

### • Trial objectives

### Primary objective:

To confirm the effectiveness of prophylactic treatment with KN057 in patients with hemophilia A with inhibitor (HAW) and hemophilia B with inhibitor (HBW).

### Secondary objectives:

In patients with HAW and HBW: 1) to evaluate the effect of prophylactic treatment with KN057 in improving the quality of life; 2) to evaluate the safety of KN057; 3) to evaluate changes in plasma concentration and pharmacodynamic indicators of KN057; and 4) to evaluate the immunogenicity of KN057.

### • Trial population and sample size

51 male patients aged 12 years-70 years (inclusive) with HAW and HBW.

### • Trial groups and trial protocol

| Group name | Treatment regimen |
|---|---|
| Trial group (prophylactic treatment with KN057) | Prophylactic treatment with KN057 for 52 weeks |
| Control group (non-prophylactic treatment) | On-demand treatment with a bypassing agent for 26 weeks and then prophylactic treatment with KN057 for 26 weeks |

Eligible subjects will be randomized into the trial group or the control group in a 2:1 ratio. Immediately after enrollment, the subjects in the trial group will receive prophylactic treatment with KN057 for 52 weeks. After enrollment, the subjects in the control group will receive on-demand treatment with a bypassing agent for 26 weeks and then prophylactic treatment with KN057 for 26 weeks.

The prophylactic treatment regimen for KN057 is: subcutaneous injection, once weekly. For subjects with body weight ≥ 50 kg, the drug will be administered at a dose of 1.5 mg/kg. For subjects with 40 kg ≤ body weight < 50 kg, the drug will be administered at a dose of 1.75 mg/kg. For subjects with 30 kg ≤ body weight < 40 kg, the drug will be administered at a dose of 2.0 mg/kg. If a subject experiences ≥2 treated spontaneous bleeding episodes within 8 weeks of receiving KN057, and the change in coagulation function after administration shows no safety concerns, the original dose can be escalated in increments of 0.25 mg/kg. For each subject, the dose of KN057 can be escalated twice at most.

If a subject experiences bleeding during the first 26 weeks after enrollment in the control group, he will receive on-demand treatment with the bypassing agent that he usually used before screening, including recombinant factor VIIa (rFVIIa) or human prothrombin complex concentrate (PCC), and after 26 weeks, he will switch to prophylactic treatment with KN057 (the subject must discontinue PCC within at least 96 hours before switching to KN057).

If a subject experiences bleeding during prophylactic treatment with KN057, rFVIIa will be administered as the preferred agent to stop bleeding; the expected minimum hemostatic dose of PCC can be used only if other effective hemostatic drugs are unavailable.

### • Trial duration

The duration of the trial is 59 weeks, including a 3-week screening period, a 26-week main trial period, a 26-week extension period, and a 4-week follow-up period following the last dose.

After receiving prophylactic treatment with KN057 for 26 weeks (main trial), the subjects in the trial group will continue to receive prophylactic treatment with KN057 for 26 weeks (extension period). After receiving non-prophylactic treatment for 26 weeks (main trial), the subjects in the control group will receive prophylactic treatment with KN057 for 26 weeks (extension period). After the main trial + the extension period, the subjects will enter the follow-up period following the last dose.

### • Trial endpoints

### Primary endpoints:

At week 26 of treatment, the annualized bleeding rates (ABRs) calculated for the trial group and the control group based on treated spontaneous and traumatic bleeding.

### Secondary endpoints:

1) At week 26 of treatment, the different types of ABR of the trial group and the control group (calculated based on bleeding^{&}, treated^{#} spontaneous bleeding, treated^{#} joint bleeding, and treated^{#} target joint^{*} bleeding, respectively).
2) At weeks 27-52 of treatment, the ABRs of the control group (calculated based on bleeding^{&}, treated^{#} bleeding^{&}, treated^{#} spontaneous bleeding, treated^{#} joint bleeding, and treated^{#} target joint^{*} bleeding, respectively).
3) At week 52 of treatment, the different types of ABR of the trial group (calculated based on bleeding^{&}, treated^{#} bleeding^{&}, treated^{#} spontaneous bleeding, treated^{#} joint bleeding, and treated^{#} target joint^{*} bleeding, respectively).
4) At weeks 26 and 52 of treatment, the proportion of subjects experiencing no treated bleeding in the trial group.
5) At weeks 1-26 and 27-52 of treatment, the proportion of subjects experiencing no treated bleeding in the control group.
6) At week 26 of treatment, changes from baseline in hemophilia joint health score (HJHS) results in the trial group and the control group.
7) At week 52 of treatment, changes from baseline in HJHS results in the trial group, and changes in HJHS results in the control group compared to those at week 26 of treatment.
8) At week 26 of treatment, changes from baseline in EuroQol five dimensions five level questionnaire (EQ-5D-5L) results in the trial group and the control group.
9) At week 52 of treatment, changes from baseline in EQ-5D-5L results in the trial group, and changes in EQ-5D-5L results in the control group compared to those at week 26 of treatment.
10) At week 26 of treatment and the end of the study, the incidence and severity of treatment emergent adverse events (TEAEs), TEAEs related to the trial drug, serious adverse events (SAEs), thromboembolic events^{@}, thrombotic microangiopathy, disseminated intravascular coagulation, allergic reactions (excluding injection site reactions), and injection site reactions in the trial group and the control group.
11) At week 26 of treatment and the end of the study, the incidence and severity of abnormalities in various laboratory safety indicators, as well as changes in safety indicators in electrocardiograms, vital signs, and physical examinations, in the trial group and the control group.
12) KN057 plasma concentration.
13) The content of total TFPI, free TFPI, and prothrombin fragment 1+2 (PF1+2) and changes from baseline.
14) The patient-level positivity rate and the sample-level positivity rate for anti-KN057 antibodies (ADAs) and neutralizing antibodies (Nabs).

Note: ^{&}Bleeding includes spontaneous and/or traumatic bleeding, and does not include surgery/operation-related bleeding; ^{#}treated bleeding means that hemostatic treatment with a bypassing agent and/or a coagulation factor is performed for this bleeding episode; ^{*}target joint refers to a joint with ≥3 spontaneous bleeding episodes within 6 months prior to screening.
^{@}Thromboembolic events include acute coronary syndrome, acute ischemic stroke, venous thrombosis, pulmonary thromboembolism, and other thromboembolic events of significant clinical significance (excluding superficial thrombophlebitis related to venous access devices). It is necessary to perform a comprehensive assessment based on symptoms, signs, and laboratory tests and confirm the diagnosis by imaging examinations (color Doppler ultrasound, CT, magnetic resonance, etc.).

### • Inclusion criteria

All patients must meet all of the following criteria to be eligible:
(1) Male, aged 12 years-70 years (inclusive) at the time of providing informed consent, with body weight ≥ 30 kg and BMI < 28 kg/m² at screening.
(2) Positive for FVIII or FIX inhibitors with a high titer (≥5 BU/mL) during the screening period; or positive for FVIII or FIX inhibitors with a low titer (0.6 BU/mL or upper limit of normal < inhibitor titer < 5 BU/mL) during the screening period, and treatment with a bypassing agent (rFVIIa or PCC) has been started.
(3) ≥6 bleeding episodes (spontaneous and/or traumatic, excluding surgery/operation-related bleeding) treated with drugs (including bypassing agents, plasma products, FVIII or FIX products, and FX activators) within 26 weeks prior to screening.
(4) No prior use of TFPI antibody-based drugs.
(5) Able and willing to undergo washout periods for the original therapeutic agents for hemophilia: a washout period of at least 48 hours for rFVIIa, a washout period of at least 72 hours for FVIII products (short-acting), a washout period of at least 96 hours for PCC, FIX products (short-acting), cryoprecipitates, or fresh frozen plasma, and a washout period of at least 5 drug half-lives for other drugs or clinical trial drugs that have relatively long half-lives, such as a washout period of at least 20 days for eftrenonacog α for injection (a recombinant human factor IX-Fc fusion protein for injection).
(6) Able to understand and comply with the requirements of the trial and willing to accept the trial.

### Example 4. Randomized, Open-Label Study for Evaluating Effectiveness and Safety of Prophylactic Treatment with KN057 Injectable Solution in Patients with Hemophilia A and Hemophilia B Without Inhibitor

### • Trial objectives

### Primary objective:

To confirm the effectiveness of prophylactic treatment with KN057 in patients with hemophilia A without inhibitor (HA) and hemophilia B without inhibitor (HB).

### Secondary objectives:

In patients with HA and HB: 1) to evaluate the effectiveness of KN057 in preventing various types of bleeding events; 2) to evaluate the effect of prophylactic treatment with KN057 in improving the quality of life; 3) to evaluate the safety of KN057; 4) to evaluate changes in plasma concentration and pharmacodynamic indicators of KN057; and 5) to evaluate the immunogenicity of KN057.

### • Trial population and sample size

123 male patients aged 12 years-70 years (inclusive) with HA and HB.

### • Trial groups and trial protocol

| **Trial part** | **Mode of enrollment** | **Group name** | **Number of subjects** | **Treatment regimen** |
|---|---|---|---|---|
| **Part A** | Random | Group 1: trial group | 34 | Prophylactic treatment with KN057 for 52 weeks |
| | | Group 2: control group | 17 | First 26 weeks: on-demand treatment with FVIII or FIX |
| | | | | Last 26 weeks: prophylactic treatment with KN057 |
| **Part B** | Non-random | Group 3: prevention group | 72 | First 26 weeks: prophylactic treatment with FVIII or FIX |
| | | | | Last 26 weeks: prophylactic treatment with KN057 |

Subjects eligible for part A will be randomized into the trial group (group 1) or the control group (group 2) in a 2:1 ratio. The trial group will receive prophylactic treatment with KN057 for 52 weeks. The control group will receive on-demand treatment with a standard half-life factor VIII (FVIII)/factor IX (FIX) product for 26 weeks and then prophylactic treatment with KN057 for 26 weeks.

Subjects eligible for part B will be enrolled in the prevention group (group 3) and receive standard prophylactic treatment with a coagulation factor (regimen: according to assessments, the investigator decides that subjects continue to use the original prophylactic treatment regimen; or standard half-life FVIII 25 IU/kg, three times weekly [HA subjects], and standard half-life FIX 40 IU/kg, twice weekly [HB subjects]) for 26 weeks and then prophylactic treatment with KN057 for 26 weeks.

The prophylactic treatment regimen for KN057 is: subcutaneous injection, once weekly. For subjects with body weight ≥ 50 kg, the drug will be administered at a dose of 1.5 mg/kg. For subjects with 40 kg ≤ body weight < 50 kg, the drug will be administered at a dose of 1.75 mg/kg. For subjects with 30 kg ≤ body weight < 40 kg, the drug will be administered at a dose of 2.0 mg/kg. If a subject experiences ≥2 treated spontaneous bleeding episodes within 8 weeks of receiving KN057, and the change in coagulation function after administration shows no safety concerns, the original dose can be escalated in increments of 0.25 mg/kg. For each subject, the dose of KN057 can be escalated twice at most.

All subjects can receive on-demand treatment with a standard half-life FVIII/FIX product if experiencing bleeding during prophylactic treatment.

### • Trial duration

The duration of the trial is 59 weeks. Part A includes a 3-week screening period, a 26-week main trial period, a 26-week extension period, and a 4-week follow-up period. Part B includes a 3-week screening period, a 26-week factor prevention period, a 26-week KN057 prevention period, and a 4-week follow-up period.

After receiving prophylactic treatment with KN057 for 26 weeks (main trial period), the subjects in the part A trial group will continue to receive prophylactic treatment with KN057 for 26 weeks (extension period). After receiving on-demand treatment for 26 weeks (main trial period), the subjects in the part A control group will receive prophylactic treatment with KN057 for 26 weeks (extension period). After receiving standard prophylactic treatment with a coagulation factor for 26 weeks (factor prevention period), the part B subjects will receive prophylactic treatment with KN057 for 26 weeks (KN057 prevention period). All subjects will be followed up for 4 weeks (follow-up period) after the last dose.

### • Trial endpoints

### Primary endpoints:

1) Part A: At week 26 of treatment, the superiority of the trial group over the control group in annualized bleeding rate (ABR, based on treated spontaneous and traumatic bleeding).
2) Part B: The non-inferiority of the KN057 prevention period to the factor prevention period in ABR (based on treated spontaneous and traumatic bleeding).

### Secondary endpoints:

### In part A:

1) At week 26 of treatment, a comparison of the ABRs of the trial group and the control group (based on bleeding^{&}, treated^{#} spontaneous bleeding, treated^{#} joint bleeding, and treated^{#} target joint^{*} bleeding, respectively).
2) At weeks 27-52 of treatment, the ABRs of the control group (based on bleeding^{&}, treated# bleeding^{&}, treated^{#} spontaneous bleeding, treated^{#} joint bleeding, and treated^{#} target joint^{*} bleeding, respectively) and a comparison with those at weeks 1-26.
3) At week 52 of treatment, the ABRs of the trial group (based on bleeding^{&}, treated# bleeding^{&}, treated^{#} spontaneous bleeding, treated^{#} joint bleeding, and treated^{#} target joint^{*} bleeding, respectively).
4) At weeks 26 and 52 of treatment, the proportion of subjects without treated bleeding in the trial group.
5) At weeks 1-26 and 27-52 of treatment, the proportion of subjects without treated bleeding in the control group.
6) At weeks 1-26 and 27-52 of treatment, the annual usage of drugs for on-demand treatment in the trial group and the control group (adjusted according to body weight).
5) At weeks 27-52 of treatment, the annual usage of drugs for on-demand treatment in the control group (adjusted according to body weight).
7) At week 26 of treatment, changes from baseline in HJHS results in the trial group and the control group.
8) At week 52 of treatment, changes from baseline in HJHS results in the trial group, and changes in HJHS results in the control group compared to those at week 26 of treatment.
9) At week 26 of treatment, changes from baseline in EQ-5D-5L results in the trial group and the control group.
10) At week 52 of treatment, changes from baseline in EQ-5D-5L results in the trial group, and changes in EQ-5D-5L results in the control group compared to those at week 26 of treatment.
11) At week 26 of treatment and the end of the study, the incidence and severity of TEAEs, TEAEs related to the trial drug, SAEs, thromboembolic events^{@}, thrombotic microangiopathy, disseminated intravascular coagulation, allergic reactions (excluding injection site reactions), and injection site reactions in the trial group and the control group.
12) At week 26 of treatment and the end of the study, the incidence and severity of abnormalities in various laboratory safety indicators, as well as changes in safety indicators in electrocardiograms, vital signs, and physical examinations, in the trial group and the control group.
13) KN057 plasma concentration.
14)The content of free total TFPI, free TFPI, and PF1+2 and changes from baseline.
15) The patient-level positivity rate and the sample-level positivity rate for ADAs and Nabs.

### In part B:

1) A comparison of the ABRs during the KN057 prevention period and the factor prevention period (based on bleeding^{&}, treated^{#} spontaneous bleeding, treated^{#} joint bleeding, and treated^{#} target joint^{*} bleeding, respectively)
2) The proportion of subjects without treated bleeding during the KN057 prevention period and the factor prevention period.
3) Changes in HJHS results during the factor prevention period (26 weeks) compared to those at the time of enrollment; changes in HJHS results during the KN057 prevention period (52 weeks) compared to those during the factor prevention period (26 weeks) and those at the time of enrollment.
4) Changes in EQ-5D-5L results during the factor prevention period (26 weeks) compared to those at the time of enrollment; changes in EQ-5D-5L results during the KN057 prevention period (52 weeks) compared to those during the factor prevention period (26 weeks) and those at the time of enrollment.
5) During the study, the incidence and severity of TEAEs, TEAEs related to the trial drug, SAEs, thromboembolic events^{@}, thrombotic microangiopathy, disseminated intravascular coagulation, allergic reactions (excluding injection site reactions), and injection site reactions.
6) During the study, the incidence and severity of abnormalities in various laboratory safety indicators, as well as changes in safety indicators in electrocardiograms, vital signs, and physical examinations.
7) KN057 plasma concentration.
8) Changes in the content of total TFPI, free TFPI, and PF1+2
9) The patient-level positivity rate and the sample-level positivity rate for ADAs and Nabs.

### • Inclusion criteria

All patients must meet all of the following criteria to be eligible:
(1) Male, aged 12 years-70 years (inclusive) at the time of providing informed consent, with body weight ≥ 30 kg and BMI < 28 kg/m² at screening.
(2) Severe and moderate-to-severe hemophilia A or hemophilia B (FVIII or FIX activity level ≤ 2%; part B can accept previous formal test results).
(3) During the screening period, negative (<0.6 BU/mL) for FVIII or FIX inhibitors, or the inhibitor level is lower than the lower limit of laboratory normal.
(4) No history of FVIII or FIX inhibitors; or the subject had inhibitors, but it has been at least 5 years since the subject successfully underwent immune tolerance induction (ITI), and the subject has not experienced recurrence of inhibitors (positive for inhibitors after successful ITI).
(5) Use of coagulation factor replacement therapy for no less than 100 exposure days prior to screening.
(6) No prior use of TFPI antibody-based drugs, such as marstacimab (Pfizer) and concizumab (Novo Nordisk).
(7) Able and willing to undergo washout periods for the original therapeutic agents for hemophilia: a washout period of at least 72 hours for FVIII products (standard half-life), a washout period of at least 96 hours for FIX products (standard half-life), prothrombin complex concentrate, cryoprecipitates, or fresh frozen plasma, and a washout period of at least 5 drug half-lives for other drugs or clinical trial drugs that have relatively long half-lives, such as a washout period of at least 20 days for eftrenonacog α for injection (a recombinant human factor IX-Fc fusion protein for injection).
(8) Able to understand and comply with the requirements of the trial and willing to accept the trial.

### Patients entering part A must also meet all of the following criteria:

(1) ≥6 bleeding episodes (spontaneous and/or traumatic, excluding surgery/operation-related bleeding) treated with drugs (including plasma products, FVIII or FIX products, prothrombin complex concentrate, and FX activators) within 26 weeks prior to screening.
(2) The subject has been undergoing on-demand treatment or non-standard prophylactic treatment or standard prophylactic treatment prior to screening, and the treatment course has not exceeded 12 weeks.

Patients entering part B must also meet one of the following criteria:
(1) The subject has been undergoing standard prophylactic treatment prior to screening, and the treatment course has exceeded 12 weeks (standard prophylactic treatment means that the compliance with the predetermined prevention regimen reaches at least 80%).
(2) After the end of enrollment in part A of the study, the subject is deemed suitable for receiving prophylactic treatment with a factor according to the investigator's assessment and has an FVIII or FIX activity level of ≤2% during the screening period.

### Example 5. In Vitro Pharmacodynamic Study of KN057 in Plasma from Patients with Hemophilia

### • Trial objective

To obtain the effective concentration range of KN057 in plasma from patients with hemophilia (type A and type B, including the types with inhibitor and the types without inhibitor; von Willebrand disease) based on the results of an *in vitro* pharmacodynamic study.

### • Trial design

The study plans to screen for 31 fasting blood samples from hemophilia patients that meet the requirements for an *in vitro* pharmacodynamic trial. The hemophilia patients include 10 patients with hemophilia A (HA), 6 patients with hemophilia B (HB), 6 patients with hemophilia A with inhibitor (HAW), 3 patients with hemophilia B with inhibitor (HBW), and 6 patients with von Willebrand disease (VWD) type 3 (given the particularity of VWD patients, the number of patients can be adjusted according to the actual clinical situation and is tentatively set to 6). About 20 mL of fasting whole blood (using a 3.2% sodium citrate anticoagulation tube) will be collected from each eligible blood-donating patient and centrifuged at the study center to obtain platelet-poor plasma (PPP), which will then be sent to a third-party laboratory for an *in vitro* pharmacodynamic trial.

The plasma samples from the hemophilia patients will be treated *in vitro* with different concentrations of KN057 or other same-target drugs that are undergoing clinical trials. The *in vitro* efficacy of KN057 will be assessed by determining the diluted prothrombin time (dPT) in the plasma samples and performing a thrombin generation assay (TGA), and comparison will be performed to find the difference in *in vitro* efficacy between KN057 and the same-target drugs.

### • Sample size

29 subjects were actually enrolled, and 29 subjects were analyzed. The specific distribution is shown in the table below.

| Patient type | HA | HB | HAW | HBW | VWD3 |
|---|---|---|---|---|---|
| Expected number of subjects enrolled | 10 | 6 | 6 | 3 | 6 (adjustable) |
| Actual number of subjects enrolled | 10 | 6 | 6 | 3 | 4 |

### • Inclusion criteria

Blood-donating patients must meet all of the following criteria (for 2 or 3, meeting one of them is sufficient) to be eligible:
1) Aged ≥18 years.
2) Severe (factor VIII or IX activity level < 1 IU/dL; when the result is ≥1 IU/dL, previous documented test results showing factor VIII or IX activity level < 1 IU/dL are acceptable) HA or HB;
   blood-donating patients without factor VIII or IX inhibitors must also meet the following inclusion criteria: no inhibitor is detected, or there are documented test results (test results issued within the past month are acceptable) showing that the blood-donating patients have no inhibitors;
   blood-donating patients with factor VIII or IX inhibitors must also meet the following inclusion criterion: positive inhibitor test results (≥0.6 BU/mL; documented test results issued within the past six months are acceptable).
3) Blood-donating patients with von Willebrand disease (blood-donating patients with VWD type 3 only): the blood-donating patients' plasma VWF antigen (VWF:Ag) levels and factor VIII activity levels must be obtained.
4) Willing to provide informed consent prior to the trial.

### • In vitro efficacy results

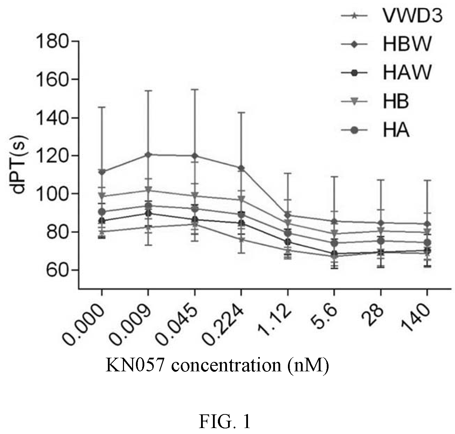

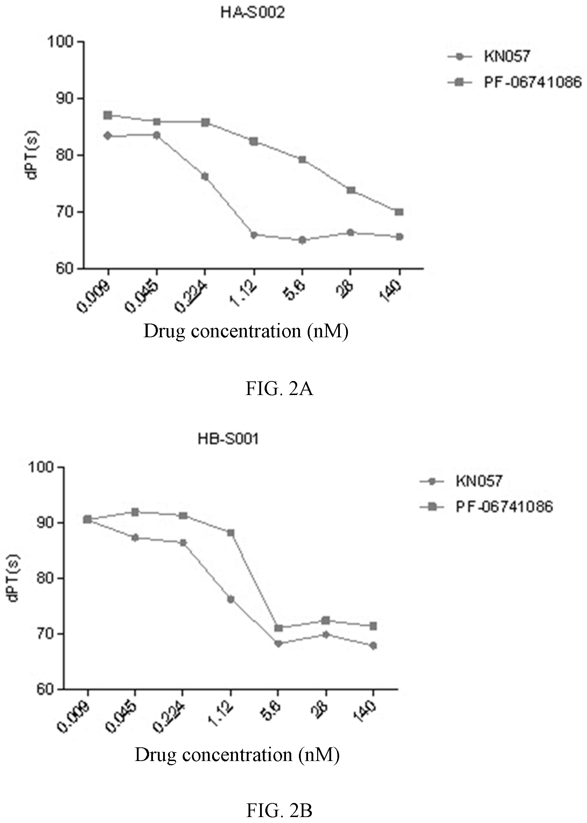

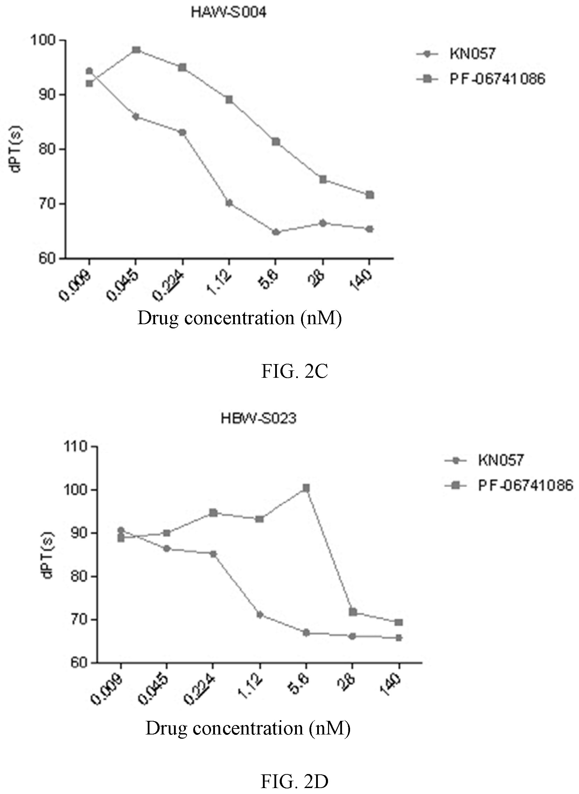

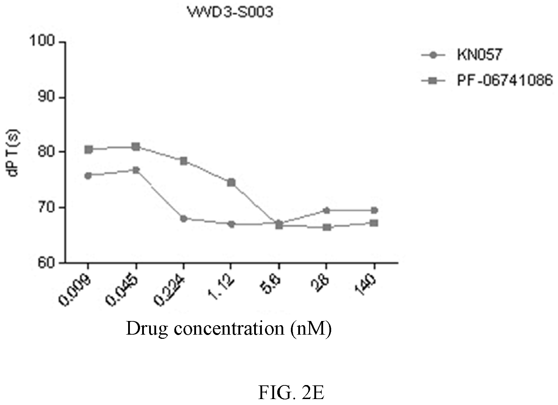

The dPT results show that:
a) After plasma from patients with hemophilia (HA, HB, HAW, and HBW) was treated *in vitro* with KN057, there was no significant change in plasma dPT when the concentration was at 0-0.224 nM, there was a significant decrease in dPT when the concentration reached 1.12 nM, dPT was decreased to the greatest extent when the concentration reached 5.6 nM, and dPT reached a plateau when the concentration was at 5.6-140 nM, suggesting that KN057 caused a concentration-dependent decrease in dPT in plasma from hemophilia patients, and the trends of effect and effective concentration ranges of KN057 in plasma from patients with various types of hemophilia were substantially consistent. The effect of KN057 in plasma from VWD3 patients was similar to that in plasma from hemophilia patients (as shown in FIG. 1).
b) The trends of effect of KN057 on dPT in plasma from patients with hemophilia without inhibitor (HA and HB) were substantially consistent with those in plasma from patients with hemophilia with inhibitor (HAW and HBW), and there was no significant difference in dPT-decreasing effect between KN057 (5.6 nM) and KN057 (28 nM).
c) A comparison of the effects of KN057 and PF-06741086 (at the same molar concentration) on dPT in plasma from patients with different types of hemophilia shows that the dPT-decreasing effect of KN057 was stronger than that of PF-06741086 (as shown in FIGs. 2A-2E).

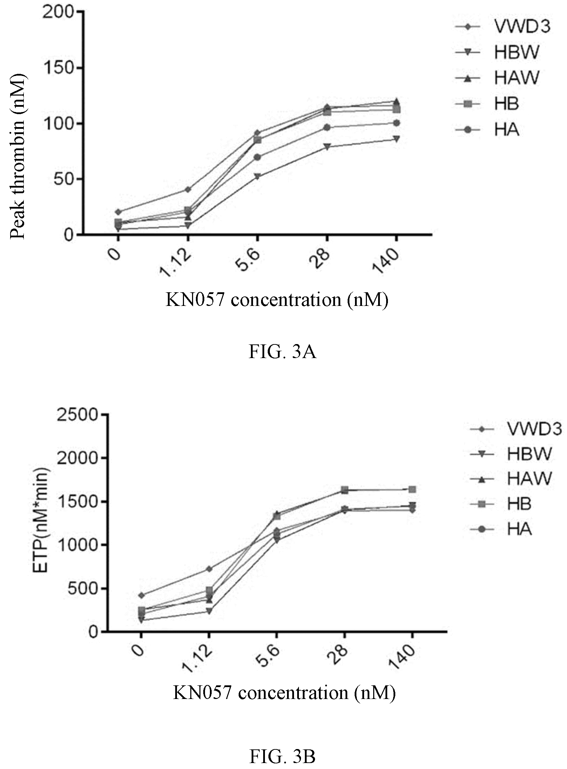

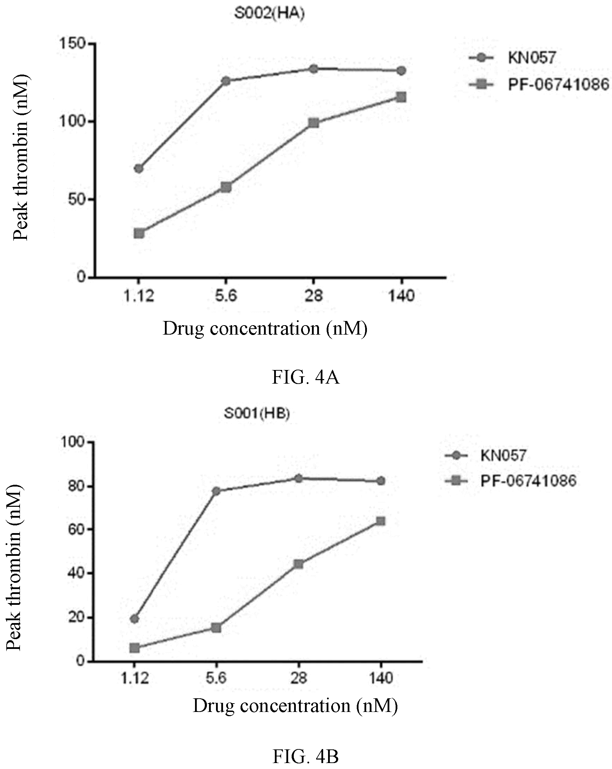

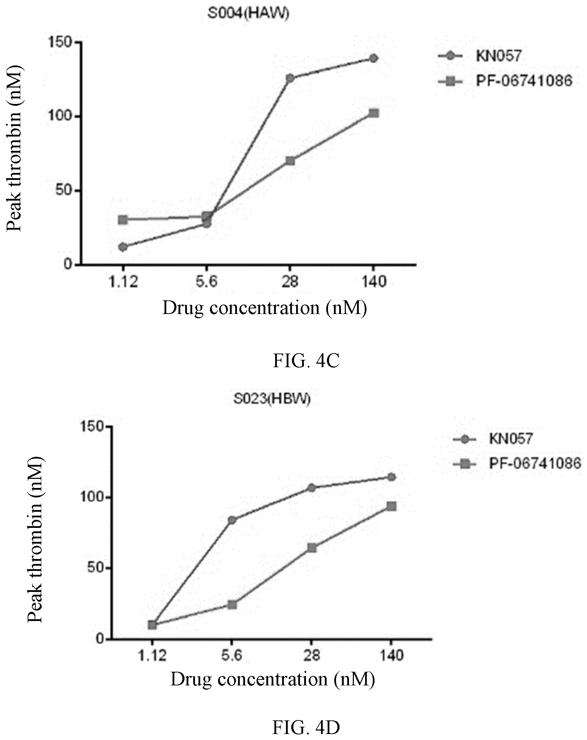

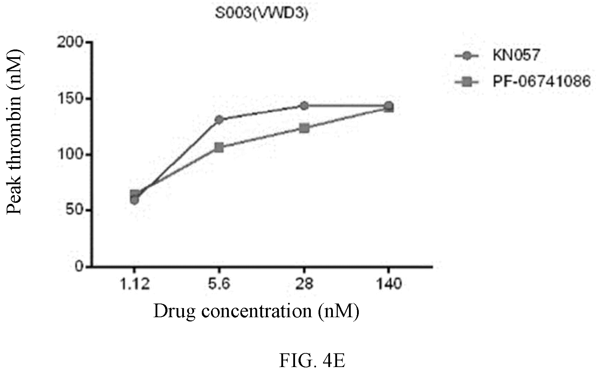

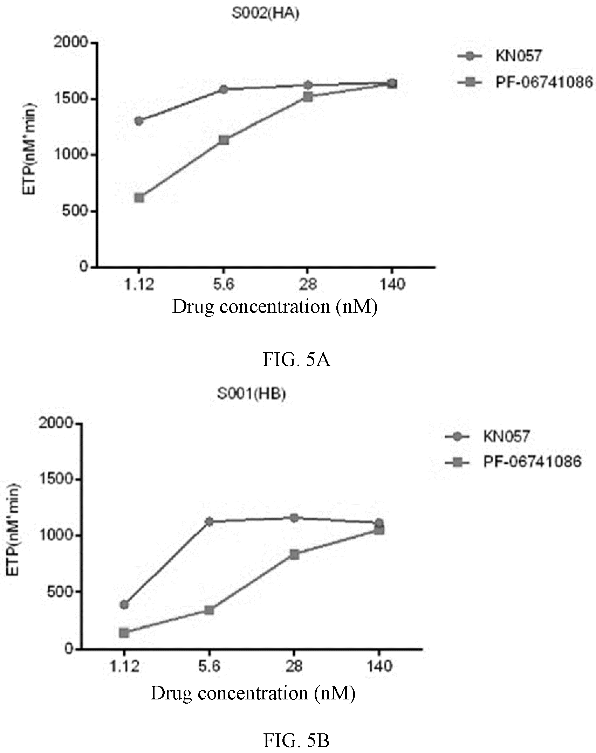

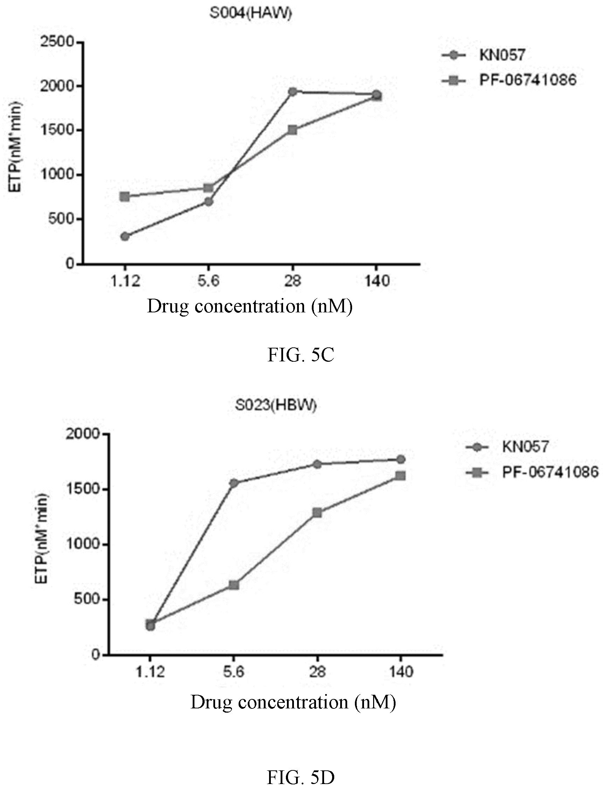

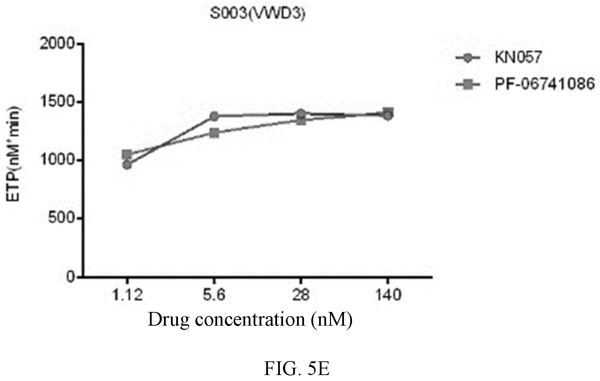

The TGA results show that:
a) In plasma from patients with various types of hemophilia, KN057 exhibited a significant peak thrombin and ETP-increasing effect when the concentration was at 1.12-5.6 nM, the effect started to flatten out when the concentration was at 5.6-28 nM, a peak was substantially reached when the concentration reached 28 nM, the average peak thrombin levels were all between 85 nM and 120 nM, and the average peak ETP levels were all between 1400 nM*min and 1650 nM*min, suggesting that KN057 caused a concentration-dependent increase in thrombin generation in plasma from hemophilia patients, and the trends of effect and effective concentration ranges of KN057 in plasma from patients with various types of hemophilia were substantially consistent. The effect of KN057 in plasma from VWD3 patients was similar to that in plasma from hemophilia patients (as shown in FIGs. 3A and 3B).
b) The trends of effect of KN057 on thrombin generation in plasma from patients with hemophilia without inhibitor (HA and HB) were consistent with those in plasma from patients with hemophilia with inhibitor (HAW and HBW), and there was no significant difference in peak thrombin and ETP-increasing effect between KN057 (5.6 nM) and KN057 (28 nM).
c) A comparison of the effects of KN057 and PF-06741086 (at the same molar concentration) on thrombin generation in plasma from patients with different types of hemophilia shows that the peak thrombin and ETP-increasing effect of KN057 was stronger than that of PF-06741086 (as shown in FIGs. 4A-4E and FIGs. 5A-5E).

SEQ ID NO: 1 (KN057 VH) 
QVQLVQSGAEVKKPGASVKVSCKASGYSFTSYGISWVKQAPGQGLEWIG
EIYPRSTNTYYNEKFMGKATLTADKSSSTAYMELSSLRSEDTAVYYCARES
FYGDYGAMDFWGQGTLVTVSS
SEQ ID NO: 2 (KN057 VH CDR1 Kabat)
   SYGIS
SEQ ID NO: 3 (KN057 VH CDR2 Kabat)
   EIYPRSTNTYYNEKFMG
SEQ ID NO: 4 (KN057 VH CDR3 Kabat)
   ESFYGDYGAMDF
SEQ ID NO: 5 (KN057 VL) 
DIVLTQSPLSLPVTPGEPASIFCKSSQSLLESDGKTYLNWLLQKPGQSPQRL
IYLVSKLDSGVPDRFSGSGSGTDFTLKISRVEAEDVGVYYCSQGTHFPRTF
GQGTKVEIK
SEQ ID NO: 6 (KN057 VL CDR1 Kabat)
   KSSQSLLESDGKTYLN
SEQ ID NO: 7 (KN057 VL CDR2 Kabat)
   LVSKLDS
SEQ ID NO: 8 (KN057 VL CDR3 Kabat)
   SQGTHFPRT
SEQ ID NO: 9 (IgG4 constant region) 
ASTKGPSVFPLAPCSRSTSESTAALGCLVKDYFPEPVTVSWNSGALTSGVH
TFPAVLQSSGLYSLSSVVTVPSSSLGTKTYTCNVDHKPSNTKVDKRVESKY
GPPCPPCPAPEFLGGPSVFLFPPKPKDTLMISRTPEVTCVVVDVSQEDPEV
QFNWYVDGVEVHNAKTKPREEQFNSTYRVVSVLTVLHQDWLNGKEYK
CKVSNKGLPSSIEKTISKAKGQPREPQVYTLPPSQEEMTKNQVSLTCLVKG
FYPSDIAVEWESNGQPENNYKTTPPVLDSDGSFFLYSRLTVDKSRWQEGN
VFSCSVMHEALHNHYTQKSLSLSLGK
SEQ ID NO: 10 (Igκ constant region)
SEQ ID NO: 11 (KN057 HC) 
QVQLVQSGAEVKKPGASVKVSCKASGYSFTSYGISWVKQAPGQGLEWIG
EIYPRSTNTYYNEKFMGKATLTADKSSSTAYMELSSLRSEDTAVYYCARES
 
FYGDYGAMDFWGQGTLVTVSSASTKGPSVFPLAPCSRSTSESTAALGCLV
KDYFPEPVTVSWNSGALTSGVHTFPAVLQSSGLYSLSSVVTVPSSSLGTKT
YTCNVDHKPSNTKVDKRVESKYGPPCPPCPAPEFLGGPSVFLFPPKPKDTL
MISRTPEVTCVVVDVSQEDPEVQFNWYVDGVEVHNAKTKPREEQFNSTY
RVVSVLTVLHQDWLNGKEYKCKVSNKGLPSSIEKTISKAKGQPREPQVYT
LPPSQEEMTKNQVSLTCLVKGFYPSDIAVEWESNGQPENNYKTTPPVLDS
DGSFFLYSRLTVDKSRWQEGNVFSCSVMHEALHNHYTQKSLSLSLGK
SEQ ID NO: 12 (KN057 LC) 
DIVLTQSPLSLPVTPGEPASIFCKSSQSLLESDGKTYLNWLLQKPGQSPQRL
IYLVSKLDSGVPDRFSGSGSGTDFTLKISRVEAEDVGVYYCSQGTHFPRTF
GQGTKVEIKRTVAAPSVFIFPPSDEQLKSGTASVVCLLNNFYPREAKVQW
KVDNALQSGNSQESVTEQDSKDSTYSLSSTLTLSKADYEKHKVYACEVT
HQGLSSPVTKSFNRGEC
SEQ ID NO: 13 (huTFPI)

MIYTMKKVHALWASVCLLLNLAPAPLNADSEEDEEHTIITDTELPPLKLM
HSFCAFKADDGPCKAIMKRFFFNIFTRQCEEFIYGGCEGNQNRFESLEECK
KMCTRDNANRIIKTTLQQEKPDFCFLEEDPGICRGYITRYFYNNQTKQCE
RFKYGGCLGNMNNFETLEECKNICEDGPNGFQVDNYGTQLNAVNNSLTP
QSTKVPSLFEFHGPSWCLTPADRGLCRANENRFYYNSVIGKCRPFKYSGC
GGNENNFTSKQECLRACKKGFIQRISKGGLIKTKRKRKKQRVKIAYEEIFV
KNM

Although specific embodiments of the present disclosure are described above, it will be appreciated by those skilled in the art that these are only illustrative and that many alterations or modifications can be made to these embodiments without departing from the principles and spirit of the present disclosure. Therefore, the scope of protection of the present disclosure is defined by the appended claims.

## Claims

1. A method for treating hemophilia, comprising a step of administering to a subject in need thereof an antibody against tissue factor pathway inhibitor (TFPI) or an antigen-binding fragment thereof, wherein the antibody comprises a heavy chain variable region and a light chain variable region;
the heavy chain variable region comprises: a VH CDR1 comprising the amino acid sequence set forth in SEQ ID NO: 2, a VH CDR2 comprising the amino acid sequence set forth in SEQ ID NO: 3, and a VH CDR3 comprising the amino acid sequence set forth in SEQ ID NO: 4;
the light chain variable region comprises: a VL CDR1 comprising the amino acid sequence set forth in SEQ ID NO: 6, a VL CDR2 comprising the amino acid sequence set forth in SEQ ID NO: 7, and a VL CDR3 comprising the amino acid sequence set forth in SEQ ID NO: 8.

2. The method according to claim 1, wherein the heavy chain variable region comprises the amino acid sequence set forth in SEQ ID NO: 1.

3. The method according to claim 1 or 2, wherein the light chain variable region comprises the amino acid sequence set forth in SEQ ID NO: 5.

4. The method according to any one of claims 1-3, wherein the antibody comprises a heavy chain that further comprises a constant region of human IgG4 or a variant thereof, e.g., the amino acid sequence set forth in SEQ ID NO: 9.

5. The method according to any one of claims 1-4, wherein the antibody comprises a light chain that further comprises a constant region of human Igκ or a variant thereof, e.g., the amino acid sequence set forth in SEQ ID NO: 10.

6. The method according to any one of claims 1-5, wherein the antibody comprises a heavy chain that comprises the amino acid sequence set forth in SEQ ID NO: 11.

7. The method according to any one of claims 1-6, wherein the antibody comprises a light chain that comprises the amino acid sequence set forth in SEQ ID NO: 12.

8. The method according to any one of claims 1-7, wherein the hemophilia is hemophilia A.

9. The method according to claim 8, wherein the hemophilia is hemophilia A with inhibitor or hemophilia A without inhibitor.

10. The method according to any one of claims 1-7, wherein the hemophilia is hemophilia B.

11. The method according to claim 10, wherein the hemophilia is hemophilia B with inhibitor or hemophilia B without inhibitor.

12. The method according to any one of claims 1-7, wherein the hemophilia is von Willebrand disease.

13. The method according to claim 12, wherein the hemophilia is von Willebrand disease type 1, type 2, or type 3.

14. The method according to any one of claims 1-13, wherein the antibody or the antigen-binding fragment thereof is administered once weekly at a dose of 1.0 mg/kg-2.0 mg/kg, as calculated based on the subject's body weight.

15. The method according to any one of claims 1-14, wherein the antibody or the antigen-binding fragment thereof is administered once weekly at a dose of 1.5 mg/kg, as calculated based on the subject's body weight.

16. The method according to any one of claims 1-14, wherein the antibody or the antigen-binding fragment thereof is administered once weekly at a dose of 1.5 mg/kg-2.0 mg/kg, as calculated based on the subject's body weight.

17. The method according to any one of claims 1-16, wherein the antibody or the antigen-binding fragment thereof is administered by subcutaneous injection.

18. The method according to claim 17, comprising subcutaneously injecting the antibody or the antigen-binding fragment thereof once weekly at a dose of 1.5 mg/kg-2.0 mg/kg, as calculated based on the subject's body weight.

19. The method according to any one of claims 1-18, wherein after the antibody or the antigen-binding fragment thereof is administered, the subject has an annualized bleeding rate of no more than 10.0, preferably no more than 8.0, more preferably no more than 5.0, and even more preferably no more than 2.0.

20. The method according to any one of claims 1-19, wherein after the antibody or the antigen-binding fragment thereof is administered, the subject has an annualized bleeding rate that decreases by no less than 60%, preferably no less than 70%, more preferably no less than 80%, and even more preferably no less than 90%, compared to that before the administration.

21. An antibody against tissue factor pathway inhibitor (TFPI) or an antigen-binding fragment thereof for treating hemophilia, wherein the antibody comprises a heavy chain variable region and a light chain variable region;
the heavy chain variable region comprises: a VH CDR1 comprising the amino acid sequence set forth in SEQ ID NO: 2, a VH CDR2 comprising the amino acid sequence set forth in SEQ ID NO: 3, and a VH CDR3 comprising the amino acid sequence set forth in SEQ ID NO: 4;
the light chain variable region comprises: a VL CDR1 comprising the amino acid sequence set forth in SEQ ID NO: 6, a VL CDR2 comprising the amino acid sequence set forth in SEQ ID NO: 7, and a VL CDR3 comprising the amino acid sequence set forth in SEQ ID NO: 8.

22. Use of an antibody against tissue factor pathway inhibitor (TFPI) or an antigen-binding fragment thereof in the preparation of a medicament for treating hemophilia, wherein the antibody comprises a heavy chain variable region and a light chain variable region;
the heavy chain variable region comprises: a VH CDR1 comprising the amino acid sequence set forth in SEQ ID NO: 2, a VH CDR2 comprising the amino acid sequence set forth in SEQ ID NO: 3, and a VH CDR3 comprising the amino acid sequence set forth in SEQ ID NO: 4;
the light chain variable region comprises: a VL CDR1 comprising the amino acid sequence set forth in SEQ ID NO: 6, a VL CDR2 comprising the amino acid sequence set forth in SEQ ID NO: 7, and a VL CDR3 comprising the amino acid sequence set forth in SEQ ID NO: 8.
